# EUROPEAN PATENT SPECIFICATION

(11) **EP 3 902 590 B1**
(45) Date of publication and mention of the grant of the patent: **07.06.2023**
(21) Application number: 19839669.9
(22) Date of filing: 17.12.2019
(51) Int. Cl.: A61M 25/00, A61M 25/04, A61M 25/10, A61M 27/00

(54) **AN IMPROVED TUBE FOR A URETHRAL CATHETER AND CATHETER COMPRISING IT**
VERBESSERTER SCHLAUCH FÜR EINEN HARNRÖHRENKATHETER UND DIESEN ENTHALTENDEN KATHETER
TUBE AMÉLIORÉ POUR CATHÉTER URÉTRAL ET CATHÉTER LE COMPRENANT

(30) Priority: 27.12.2018 PT 2018115232
(43) Date of publication of application: 03.11.2021
(73) Proprietor: Pacheco dos Santos Dias, José António, 1250-068 Lisboa (PT)
(72) Inventor: Pacheco dos Santos Dias, José António, 1250-068 Lisboa (PT)
(74) Representative: do Nascimento Gomes, Rui
(86) International application number: PCT/IB2019/060923
(87) International publication number: WO 2020/136503

(56) References cited:
- EP-A1- 2 932 996
- WO-A1-99/07420
- WO-A1-2015/200538
- CN-Y- 2 131 529
- CN-Y- 201 008 694
- US-A- 5 250 029

## Description

### FIELD OF THE INVENTION

The present invention is enclosed in the area of multi-way urethral catheters, which are suitable for draining urine or blood from the bladder of a patient.

### PRIOR ART

The formation of clots within the bladder is a major problem in medicine and, until now, has not been solved. Clot formation occurs by the presence of blood within the bladder, either in catheterised or non-catheterised patients, after surgery (urological or otherwise) or in other contexts.

The number of causes of bleeding into the bladder is varied, from very frequent and serious tumours of the bladder, kidneys or ureters, to benign diseases, also very common, such as prostate diseases, lithiasis (urinary stones), infections, other diseases of the kidneys and ureters, systemic diseases like haematological disorders and secondary to several treatments and medicines, among others. This type of haemorrhage is almost universal in the postoperative period of multiple urological surgeries.

This problem occurs both in the hospital / inpatient setting and in the outpatient setting, outside the health facility setting (i.e. with outpatients), resulting in many patients having to be treated and catheterised as a result of bleeding.

Many are the cases in which patients are catheterised because they cannot urinate, but also as many are the other medical situations where such devices are required - for example, to measure the amount of urine produced. Presently, patients are being given medications to fight the formation of thrombi (which cause strokes), such as antiplatelet agents and anticoagulants. These drugs further contribute to urinary haemorrhage and an increased risk of clot formation and clogging of catheters.

An additional, unsolved, problem is the frequent and recurrent obstruction of the catheter (catheters for bladder drainage, to drain the urine inside this organ), specifically the clogging caused by clots in these catheters, which consist of a major cause of suffering to the catheterised patient, namely severe pain and discomfort due to bladder distension. This clogging significantly increases the risk of trauma to the urinary tract and infection, by the manipulation which the clearing of the clot in the catheter requires, namely by means of vesical washing, instillation of liquid into the bladder, replacements of catheter, etc.

This seemingly simple problem consumes countless resources, both from health professionals (doctors and nurses, operating assistants) and from equipment, devices and accessories. It consists, moreover, of a cause of pronounced suffering of the affected patients (by stopping the correct emptying of the bladder and sensation of repletion that is thereby caused), with intense pain.

This problem also increases the risk of hospital infection, the length of hospital stay, and of the number and severity of associated complications. By increasing the time patients are bedridden, the number of manoeuvres and manipulations related to the catheter (such as continuous and manual washes) triggers the risk of thromboembolic complications, infectious, traumatic complications for the urinary tract and consumption of medicaments, among others.

Catheters which provide for active drainage of the bladder are known in the art, namely three-way catheters as that of the solutions disclosed in patent application US 2006/0064065, in which an instillation liquid is provided through the catheter and into the bladder, such liquid and the contents of the bladder then exiting the bladder through one or more drainage openings formed in the catheter and connected to another of the ways. Such catheters also apply the principle of the Foley catheter (as is also the case of the solutions of US 2006/0064065), in which a balloon formed in the tube of the catheter is filled, so that the tip of the catheter remains in position, inside the bladder, during operation.

However, such catheters do not provide for a correct and efficient drainage of the bladder, especially in cases of bleeding inside the bladder, that is, when, regardless of the cause, there is blood in this organ, which may lead to the formation of clots.

The multi-channel tube and catheter of the present invention, by means of the innovative and differentiating features, overcome such issues, facilitating a continuous and abundant elimination of both the liquid inside the bladder and any small clots that form in the bladder.

### SUMMARY OF THE INVENTION

It is therefore an object of the present invention a tube for a urethral catheter (1) comprising at least three channels, wherein:
- more than one instillation opening (2),
- at least one drainage opening (3), and
- a distal balloon (4),
are formed in the tube and connected to different channels, the distal balloon (4) is formed in the tip of the tube and each instillation opening (2) are arranged relatively to the distal balloon in such a way that instillation liquid exiting such opening hits directly the distal ballon (4), when the distal balloon (4) is inflated, thereby spreading through the bladder of a patient, when in operation. The relative positioning of the instillation opening (2) and the distal balloon (4), together with the fact that the distal balloon (4) is positioned in the tip of the tube, provides that instillation liquid exiting the opening is spread through the bladder, after hitting the distal balloon (4).

The tube of the present invention thereby limits urinary stasis / stagnation in the bladder, in particular of blood present therein, preventing the formation of clots and consequent obstruction of the catheter. It enables to drastically reduce the need for medical and nursing care in hospitalized patients, be it in postoperative urological surgeries or in other contexts and clinical situations.

In an advantageous configuration of the tube of the present invention, it further comprises a proximal balloon (5) which consists of a Foley type balloon, the instillation opening (2) being arranged between the two balloons. Thus, the proximal balloon (5) provides that the tip of the tube is correctly maintained in a desired position inside the bladder, while not obstructing the referred operation of the instillation opening (2).

In another inventive aspect of the tube of the present invention, the distal balloon (4) has a shape with a circumferential cross section, and the surface of the distal balloon (4) is striated by a plurality of circumferential grooves and lumps (6) arranged on such surface, the grooves and lumps (6) being longitudinally, circumferentially or spirally arranged. Such shape and striated configuration provide that instillation liquid entering the bladder acquires a swirl movement, in particular a helical movement, by hitting the grooves and lumps (6) longitudinally, circumferentially or spirally arranged in the surface. Preferably, the distal balloon (4) has a substantially conical shape and its least diameter area faces the tube. Such shape provides an enhanced swirl movement of the instillation liquid. Alternatively, and although not efficient but easier to produce, the distal balloon (4) has a spherical shape. The swirl movement allows to highly reduce stasis /stagnation in the bladder.

It is also an object of the present invention a urethral catheter (1) comprising at least three external openings (7) and a tube as of any of the described configurations, each channel of the tube being connectable to one of the external openings (7). Such catheter provides for the advantages described for the tube of the present invention.

### DESCRIPTION OF FIGURES

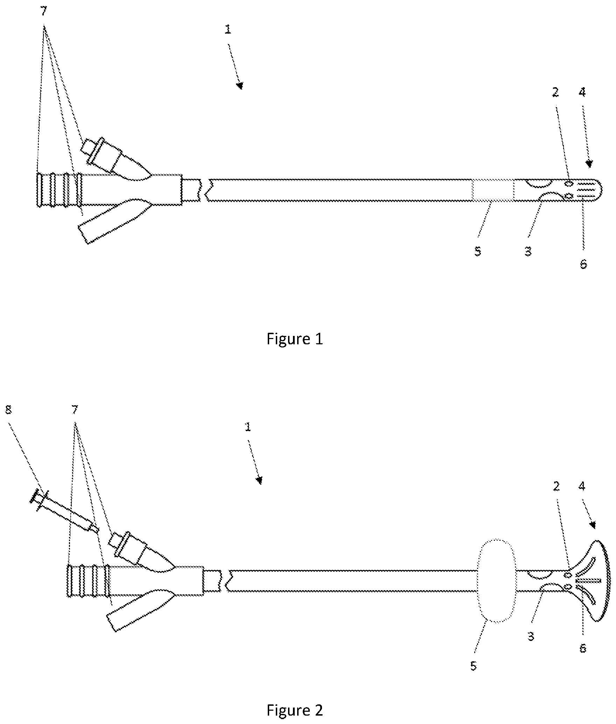

Figure 1- representation of an embodiment of the urethral catheter (1) of the present invention, comprising three external openings (7) and the tube of the present invention with three channels, each channel of the tube being connected to one of the external openings (7). The tube comprises an instillation opening (2), several drainage openings (3) and a distal balloon (4). These are connected to different channels. In Figure 1, the catheter is not presented in its entirety, which can be longer or shorter. The balloons are not filled. Although not seen in the present figure, the catheter contains a distal balloon (4) and a proximal balloon (5) (see Figure 2).
Figure 2 - representation of the same embodiment of the urethral catheter (1) as of Figure 1, in the present case in which air was inserted through the opening connected by a channel to the distal and proximal balloons (5), by means of a syringe (8), the balloons thereby being filled.

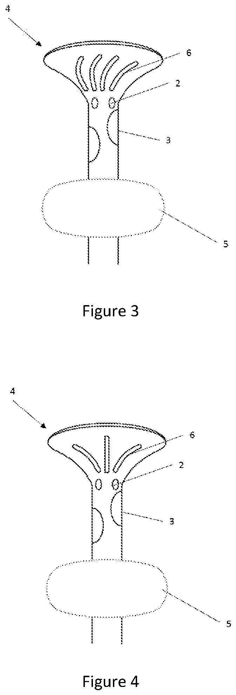

Figure 3 - representation of the distal part of the catheter / tube, containing the distal and proximal balloons (5). The distal balloon (4) which has a shape with a circumferential cross section, and the surface of the distal balloon (4) is striated by a plurality of circumferential grooves and lumps (6) arranged on such surface, the grooves and lumps (6) being spirally arranged. Such shape and striated configuration provide that instillation liquid entering the bladder acquires a swirl movement, in particular a helical movement, by hitting the grooves and lumps (6) arranged spirally in the surface. The distal balloon (4) has a substantially conical shape and its least diameter area faces the tube.
Figure 4 - representation of the distal part of the catheter / tube, containing the distal and proximal balloons (5). In this case, and with regard to Figure 3, the grooves and lumps (6) are arranged longitudinally.

### DETAILED DESCRIPTION

The more general and advantageous configurations of the present invention are described in the Summary of the invention. Such configurations are detailed below in accordance with other advantageous and/or preferred embodiments of implementation of the present invention.

In a preferred and inventive embodiment of the tube of the present invention, it comprises only three channels and the two balloons are connected to a same channel, the distal balloon (4) being inflatable at the same time or after inflation of the proximal balloon (5) through such channel. Therefore, a same channel may be used to inflate the two balloons, thus avoiding the (more complicated) formation of an additional channel (for the second balloon) in the tube, which would decrease the section of the tube available for drainage. Alternatively, and although more complicated to produce, it is also possible to provide a tube which comprises at least four channels and the two balloons are connected to two different channels, each of the two balloons thereby being independently inflatable through each of said two different channels.

Preferably, the drainage openings have an oval shape and/or are uniformly arranged/distributed between the two balloons and on the circumference of the surface of the tube. Between the balloons there may be 2 or 3 orifices. In case of two orifices, each are separated by 5 to 10 mm, on opposite sides of the catheter, with their centre 180° apart from each other (seen from a sectional view of the catheter). In case of 3 orifices, their centre are 120° apart from each other, when seen from a sectional view of the catheter. An additional opening may be located at the tip of the tube (on the centre of the catheter and of the balloon).

Preferably, the material the tube is made of consists of latex, silicone or a silicone polymer, optionally an inert silicone polymer (that is, the same materials the other standard bladder catheters are made of), or other similar materials.

Preferably, the drainage openings (3) are arranged between the two balloons, preferably each instillation opening (2) being arranged between the distal balloon (4) and the drainage openings (3). There may be an additional orifice at the tip of the catheter, within the distal balloon (4).

The catheter of the present invention may take two main configurations as regards the number of external openings (7): it comprises only three external openings (7) - which consist of two inlets and one outlet - and the tube is according to claim 6, or it comprises at least four external openings (7) - which consist of three inlets and one outlet - and the tube is according to claim 7.

In an inventive aspect of the catheter of the present invention, it comprises a tube with a distal and a proximal balloon (5), and each inlet which is connectable to the channel of the tube, which in turn is connected to the two balloons has a valve, said valve being such that it has a minimum pressure threshold which prevents any of the balloons from deflating. It thus provides a single inlet/channel for the two balloons. Alternatively, the catheter comprises a tube with a distal and a proximal balloon (5), the external openings (7) and the tube with the balloons being formed in a single piece, or the external openings (7) are separated from the tube with the balloons, and are connectable to the tube with the balloons.

As will be clear to one skilled in the art, the present invention should not be limited to the embodiments described herein, and a number of changes are possible which remain within the scope of the present invention as defined by the appended claims.

Of course, the preferred embodiments shown above are combinable, in the different possible forms, being herein avoided the repetition all such combinations.

## Claims

1. A tube for a urethral catheter (1) **characterised in that** it comprises at least three channels, wherein:
- more than one instillation opening (2),
- at least one drainage opening (3), and
- a distal balloon (4),
are formed in the tube and connected to different channels, the distal balloon (4) is formed in the tip of the tube and each instillation opening (2) is arranged relatively to the distal balloon (4) in such a way that instillation liquid exiting each instillation opening (2) hits directly the distal balloon (4), when the distal balloon (4) is inflated.

2. A tube according to the previous claim wherein it further comprises a proximal balloon (5) which consists of a Foley type balloon, the instillation opening (2) being arranged between the two balloons.

3. A tube according to any of the previous claims wherein the distal balloon (4) has a shape with a circumferential cross section, and the surface of the distal balloon (4) is striated by a plurality of grooves and lumps (6) arranged on such surface, the grooves and lumps (6) being longitudinally, circumferentially or spirally arranged.

4. A tube according to any of the claims 2-3 wherein the distal balloon (4) has a substantially conical shape and its least diameter area faces the tube.

5. A tube according to any of the claims 2-4 wherein the distal balloon (4) has a spherical shape.

6. A tube according to any of the claims 2-5 wherein it comprises only three channels and the two balloons are connected to a same channel, the distal balloon (4) being inflatable at the same time or after inflation of the proximal balloon (5) through such channel.

7. A tube according to any of the claims 2-6 wherein it comprises at least four channels and the two balloons are connected to two different channels, each of the two balloons thereby being independently inflatable through each of said two different channels.

8. A tube according to any of the claims 2-7 wherein the drainage openings have an oval shape and/or are uniformly arranged/distributed between the two balloons and on the circumference of the surface of the tube.

9. A tube according to any of the preceding claims wherein it further comprises an additional drainage opening, located in the tip of the tube.

10. A tube according to any of the preceding claims wherein the material it is made of consists of latex, silicone or a silicone polymer, optionally an inert silicone polymer.

11. A tube according to any of the claims 2-10 wherein the drainage openings (3) are arranged between the two balloons, preferably each instillation opening (2) being arranged between the distal balloon (4) and the drainage openings (3).

12. A urethral catheter (1) **characterised in that** it comprises at least three external openings (7) and a tube according to any of the preceding claims, each channel of the tube being connectable to one of the external openings (7).

13. A catheter according to the previous claim, wherein it comprises only three external openings (7) and the tube is according to claim 6, or it comprises at least four external openings (7) and the tube is according to claim 7.

14. A catheter according to any of the claims 12-13, wherein the tube is according to any of the claims 2-10 and each inlet which is connectable to the channel of the tube which in turn is connected to the two balloons has a valve, said valve being such that it has a minimum pressure threshold which prevents any of the balloons from deflating.

15. A catheter according to any of the claims 12-14 wherein the tube is according to any of the claims 2-11 and the external openings (7) and the tube with the balloons are formed in a single piece, or the external openings (7) are separated from the tube with the balloons, and are connectable to the tube with the balloons.

## Patentansprüche

1. Schlauch für einen Harnröhrenkatheter (1), **dadurch gekennzeichnet, dass** er mindestens drei Kanäle umfasst, wobei:
- eine Instillationsöffnung (2),
- mindestens eine Drainageöffnung (3), und
- einen distalen Ballon (4),
in der Röhre ausgebildet und mit verschiedenen Kanälen verbunden sind, der distale Ballon (4) in der Spitze der Schlauch ausgebildet ist und jede Instillationsöffnung (2) so angeordnet ist, dass aus diesen Öffnungen austretende Instillationsflüssigkeit direkt auf den distalen Ballon (4) trifft, wenn der distale Ballon (4) aufgeblasen wird.

2. Schlauch nach dem vorhergehenden Anspruch, wobei er ferner einen proximalen Ballon (5) umfasst, der aus einem Foley-Ballon besteht, wobei die Instillationsöffnung (2) zwischen den beiden Ballons angeordnet ist.

3. Schlauch nach einem der vorhergehenden Ansprüche, wobei der distale Ballon (4) eine Form mit einem umlaufenden Querschnitt hat und die Oberfläche des distalen Ballons (4) durch eine Vielzahl von Rillen und Klumpen (6), die auf dieser Oberfläche angeordnet sind, gestreift ist, wobei die Rillen und Klumpen (6) in Längsrichtung, in Umfangsrichtung oder spiralförmig angeordnet sind.

4. Schlauch nach einem der Ansprüche 2-3, wobei der distale Ballon (4) eine im Wesentlichen konische Form hat und sein Bereich mit dem geringsten Durchmesser dem Schlauch zugewandt ist.

5. Schlauch nach einem der Ansprüche 2-4, wobei der distale Ballon (4) eine sphärische Form hat.

6. Schlauch nach einem der Ansprüche 2-5, wobei er nur drei Kanäle umfasst und die beiden Ballons mit demselben Kanal verbunden sind, wobei der distale Ballon (4) gleichzeitig oder nach dem Aufblasen des proximalen Ballons (5) durch diesen Kanal aufblasbar ist.

7. Schlauch nach einem der Ansprüche 2-6, wobei er mindestens vier Kanäle umfasst und die beiden Ballons mit zwei verschiedenen Kanälen verbunden sind, wodurch jeder der beiden Ballons unabhängig voneinander durch jeden der beiden verschiedenen Kanäle aufblasbar ist.

8. Schlauch nach einem der vorhergehenden Ansprüche, wobei die Drainageöffnungen eine ovale Form haben und/oder gleichmäßig zwischen den beiden Ballons und auf dem Umfang der Oberfläche des Schlauchs angeordnet/verteilt sind.

9. Schlauch nach einem der vorhergehenden Ansprüche, wobei er ferner eine zusätzliche Drainageöffnung umfasst, die sich in der Spitze des Schlauchs befindet.

10. Schlauch nach einem der vorhergehenden Ansprüche, wobei das Material, aus dem er hergestellt ist, aus Latex, Silikon oder einem Silikonpolymer, gegebenenfalls einem inerten Silikonpolymer, besteht.

11. Schlauch nach einem der Ansprüche 2-10, wobei die Drainageöffnungen (3) zwischen den beiden Ballons angeordnet sind, vorzugsweise ist jede Instillationsöffnung (2) zwischen dem distalen Ballon (4) und den Drainageöffnungen (3) angeordnet.

12. Harnröhrenkatheter (1), **dadurch gekennzeichnet, dass** er mindestens drei Außenöffnungen (7) und einen Schlauch nach einem der vorhergehenden Ansprüche umfasst, wobei jeder Kanal des Schlauchs mit einer der Außenöffnungen (7) verbunden werden kann.

13. Katheter nach dem vorhergehenden Anspruch, wobei er nur drei äußere Öffnungen (7) umfasst und der Schlauch nach Anspruch 6 ist, oder er mindestens vier äußere Öffnungen (7) umfasst und der Schlauch nach Anspruch 7 ist.

14. Katheter nach einem der Ansprüche 12-13, wobei der Schlauch nach einem der Ansprüche 2-10 ist und jeder Einlass, der mit dem Kanal des Schlauchs verbunden werden kann, der wiederum mit den beiden Ballons verbunden ist, ein Ventil aufweist, wobei das Ventil so beschaffen ist, dass es eine Mindestdruckschwelle aufweist, die verhindert, dass einer der Ballons entleert wird.

15. Katheter nach einem der Ansprüche 12-14, wobei der Schlauch nach einem der Ansprüche 2-11 ist und die äußeren Öffnungen (7) und der Schlauch mit den Ballons in einem einzigen Stück ausgebildet sind, oder die äußeren Öffnungen (7) von dem Schlauch mit den Ballons getrennt sind und mit dem Schlauch mit den Ballons verbunden werden können.

## Revendications

1. Tube pour un cathéter urétral (1) **caractérisé en ce qu'**il comprend au moins trois canaux, où :
- une ouverture d'instillation (2),
- au moins une ouverture de drainage (3),et
- un ballonnet distal (4),
sont formés dans le tube et reliés à différents canaux, le ballonnet distal (4) est formé dans la pointe du tube et chaque ouverture d'instillation (2) est conçue de sorte que le liquide d'instillation sortant desdites ouvertures vienne impacter directement le ballonnet distal (4), lorsque le ballonnet distal (4) est gonflé.

2. Tube selon la revendication précédente comprenant en outre un ballonnet proximal (5) qui consiste en un ballonnet du type Foley, l'ouverture d'instillation (2) étant disposée entre les deux ballonnets.

3. Tube selon l'une quelconque des revendications précédentes où le ballonnet distal (4) présente une forme ayant une section transversale circonférentielle, et la surface du ballonnet distal (4) est striée d'une pluralité de rainures et crêtes (6) disposées sur ladite surface, les rainures et les crêtes (6) étant disposées longitudinalement, circonférentiellement ou en spirale.

4. Tube selon l'une quelconque des revendications 2 et 3 où le ballonnet distal (4) présente une forme substantiellement conique et sa zone de plus petit diamètre est tournée vers le tube.

5. Tube selon l'une quelconque des revendications 2 à 4 où le ballonnet distal (4) présente une forme sphérique.

6. Tube selon l'une quelconque des revendications 2 à 5 comprenant uniquement trois canaux et dont les deux ballonnets sont reliés à un même canal, le ballonnet distal (4) pouvant être gonflé en même temps que ou après le gonflage de ballonnet proximal (5) à travers ledit canal.

7. Tube selon l'une quelconque des revendications 2 à 6 comprenant au moins quatre canaux et dont les deux ballonnets sont reliés à deux canaux différents, chacun des deux ballonnets pouvant ainsi être gonflé séparément à travers chacun desdits deux canaux différents.

8. Tube selon l'une quelconque des revendications précédentes où les ouvertures de drainage présentent une forme ovale et/ou sont disposées/distribuées uniformément entre les deux ballonnets et sur la circonférence de la surface du tube.

9. Tube selon l'une quelconque des revendications précédentes comprenant en outre une ouverture de drainage supplémentaire, située dans la pointe du tube.

10. Tube selon l'une quelconque des revendications précédentes où le matériau dont il est fabriqué est constitué de latex, de silicone ou d'un polymère de silicone, facultativement un polymère de silicone inerte.

11. Tube selon l'une quelconque des revendications 2 à 10 où les ouvertures de drainage (3) sont disposées entre les deux ballonnets, de préférence chaque ouverture d'instillation (2) étant disposée entre le ballonnet distal (4) et les ouvertures de drainage (3).

12. Cathéter urétral (1) **caractérisé en ce qu'**il comprend au moins trois ouvertures externes (7) et un tube selon l'une quelconque des revendications précédentes, chaque canal du tube pouvant être relié à l'une des ouvertures externes (7).

13. Cathéter selon la revendication précédente comprenant uniquement trois ouvertures externes (7) et dont le tube est selon la revendication 6, ou comprenant au moins quatre ouvertures externes (7) et dont le tube est selon la revendication 7.

14. Cathéter selon l'une quelconque des revendications 12 et 13, où le tube est selon l'une quelconque des revendications 2 à 10 et chaque entrée pouvant être reliée au canal du tube qui est relié à son tour aux deux ballonnets, est dotée d'une soupape, cette soupape étant telle qu'elle a un seuil de pression minimale qui empêche que les ballonnets se dégonflent.

15. Cathéter selon l'une quelconque des revendications 12 à 14, où le tube est selon l'une quelconque des revendications 2 à 11 et les ouvertures externes (7) et le tube avec les ballonnets forment une seule pièce, ou les ouvertures externes (7) sont séparées du tube avec les ballonnets et peuvent être reliées au tube avec les ballonnets.
